# EUROPÄISCHE PATENTSCHRIFT

(11) **EP 1 060 718 B1**
(45) Veröffentlichungstag und Bekanntmachung des Hinweises auf die Patenterteilung: **10.11.2004**
(21) Anmeldenummer: 00112810.7
(22) Anmeldetag: 16.06.2000
(51) Int. Cl.: A61F 5/03, A61F 5/01

(54) **Bekleidungsstück und orthopädische Einrichtung dafür**
Garment and corresponding orthopaedic device
Vêtement et dispositif orthopédique correspondant

(30) Priorität: 18.06.1999 DE 29911206 U
(43) Veröffentlichungstag der Anmeldung: 20.12.2000
(73) Patentinhaber: Hildebrandt, Hans-Dietrich, Dr. med., 34292 Ahnatal (DE)
(72) Erfinder: Hildebrandt, Hans-Dietrich, Dr. med., 34292 Ahnatal (DE)
(74) Vertreter: Freiherr von Schorlemer, Reinfried, Dipl.-Phys.

(56) Entgegenhaltungen:
- EP-A- 0 461 319
- US-A- 3 521 623
- US-A- 3 868 952
- US-A- 4 120 297
- US-A- 4 926 502
- US-A- 5 724 993

## Beschreibung

Die Erfindung betrifft ein Bekleidungsstück aus einem flexiblen bzw. dehnbaren Material und eine orthopädische Einrichtung dafür.

Die Behandlung von Menschen, die unter Osteoporose leiden, ist bisher noch nicht in jeder Hinsicht zufriedenstellend. Die meistens bei Frauen auftretende Osteoporose besteht in einer Erkrankung des Skelettsystems durch Verminderung der Knochenmasse. Obwohl die Osteoporose unter anderem auch durch Stoffwechselerkrankungen, Inaktivität und Cortisonbehandlung verursacht werden kann, ist ihre Hauptursache in einem altersbedingten Mangel an anabolen Hormonen (Östrogenen) zu sehen, die zum Aufbau von Knochengewebe benötigt werden. Eine Folge dieses Mangels ist, daß das Knochengewebe allmählich abgebaut wird, die Knochenbälkchen dünner werden und sich schließlich auflösen und eine erhöhte Frakturneigung entsteht.

Im Bereich der Wirbelsäule führt die Osteoporose zur Ausprägung eines Hohlrundrückens. Da die Widerstandsfähigkeit der im Rundbereich belasteteten Brustwirbel gering ist, ergeben sich Wirbelbrüche, die eine Bildung von Keilwirbeln und damit eine weitere Verstärkung des Rundbereichs zur Folge haben. Im Hohlbereich werden dagegen seltener Frakturen beobachtet, weil dort überwiegend die hinten liegenden, vergleichsweise harten Wirbelbereiche belastet werden.

Zur Reduzierung oder Vermeidung der Osteoporose dienen heute überwiegend Hormon- und Medikamententherapien, um den Patienten fehlende Östrogene und andere Substanzen (z. B. Calcium, Natriumfluorid, Biphosphonate) zuzuführen, die den Knochenaufbau fördern oder den Knochenabbau vermindern. Damit diese Substanzen über den Blutweg in die Knochen gelangen und dort wirksam werden, werden diese Therapien häufig mit speziellen, die Durchblutung fördernden Gymnastiken kombiniert. Die auf diese Weise erzielbaren Heilerfolge sind jedoch unbefriedigend. Außerdem soll die Gymnastik die Muskulatur stärken und dem Hohlrundrücken entgegenwirken.

Daneben sind sogenannte Lumbosakral-Pelotten bekannt (EP 0 479 014 A1), die an Stützbandagen oder Gürteln befestigt und aus Luftkissen hergestellt werden, die eine Vielzahl von Luftkammern aufweisen, die durch teilweise unterbrochene Nähte miteinander in Verbindung stehen. Derartige Pelotten dienen dem Zweck, durch reflektorische Muskelarbeit die Bauch-, Gesäß-, und Ischiocruralmuskulatur anzuspannen und gleichzeitig die Rückenstreckmuskulatur zu detonisieren, um dadurch das Becken aufzurichten und als Folge davon eine Streckung der Lendenlordose und Brustkyphose, d. h. eine Streckung der Wirbelsäule insgesamt zu erreichen. Das Tragen einer solchen Pelotte kann zur Osteoporose-Bekämpfung so lange eingesetzt werden, wie ein Hohlrundrücken noch nicht fixiert ist, weil dann der Bildung eines Hohlrundrückens und von Wirbelbrüchen besonders im Brustwirbelbereich noch entgegengewirkt werden kann.

Mit Hilfe der bekannten Lumbosakral-Pelotte sollen somit insbesondere die Mechanorezeptoren der Lendenregion gereizt und die relevanten Muskeln aktiviert werden, um die Körperhaltung zu verbessern. Eine dadurch herbeigeführte Normalisierung des Wirbelsäulenaufbaus kann aber weder den Verlust an Knochenmasse und -architektur unterbrechen, noch langfristig die Osteoporose aufhalten oder deren wichtigste Folgeerscheinung, nämlich das Auftreten von Wirbelbrüchen, gänzlich vermeiden.

Schließlich ist es bekannt, stabile Wirbelkörperfrakturen oder vorhandene Wirbelsäulenverkrümmungen mit Hilfe von Gipskorsetts oder zu deren Ersatz geschaffenen, orthopädischen Einrichtungen ruhig zu stellen bzw. zur reduzieren (US-A-4 120 297). Der Erfindung liegt die Aufgabe zugrunde, ein Mittel zu schaffen, mit dem die Durchblutung der Knochen im Bereich der Lenden- und Brustwirbelsäule gefördert und dadurch die Wirkung der bisherigen, auf Medikamenten und Gymnastik aufbauenden Kombinationstherapien verbessert werden kann.

Zur Lösung dieser Aufgabe dient erfindungsgemäß das durch die Merkmale des Anspruchs 1 gekennzeichnete Bekleidungsstück und die im Anspruch 7 gekennzeichnete orthopädische Einrichtung.

Weitere vorteilhafte Merkmale der Erfindung ergeben sich aus den Unteransprüchen.

Die Erfindung wird nachfolgend in Verbindung mit den beiliegenden Zeichnungen und einem Ausführungsbeispiel näher erläutert. Es zeigen:
Fig. 1 grob schematisch ein erfindungsgemäßes, zur Behandlung der Osteoporose bestimmtes Bekleidungsstück.
Fig. 2 eine Draufsicht auf eine Luftkissenleiste für das Kleidungsstück nach Fig. 1 in einem etwas vergrößerten Maßstab; und
Fig. 3 und 4 Querschnitte durch eine mit dem Bekleidungsstück nach Fig. 1 verbindbare und mit Luftkissenleisten versehene orthopädische Einrichtung im Bereich der Schnittlinien III-III und IV - IV der Fig. 2 und in einem nochmals vergrößerten Maßstab.

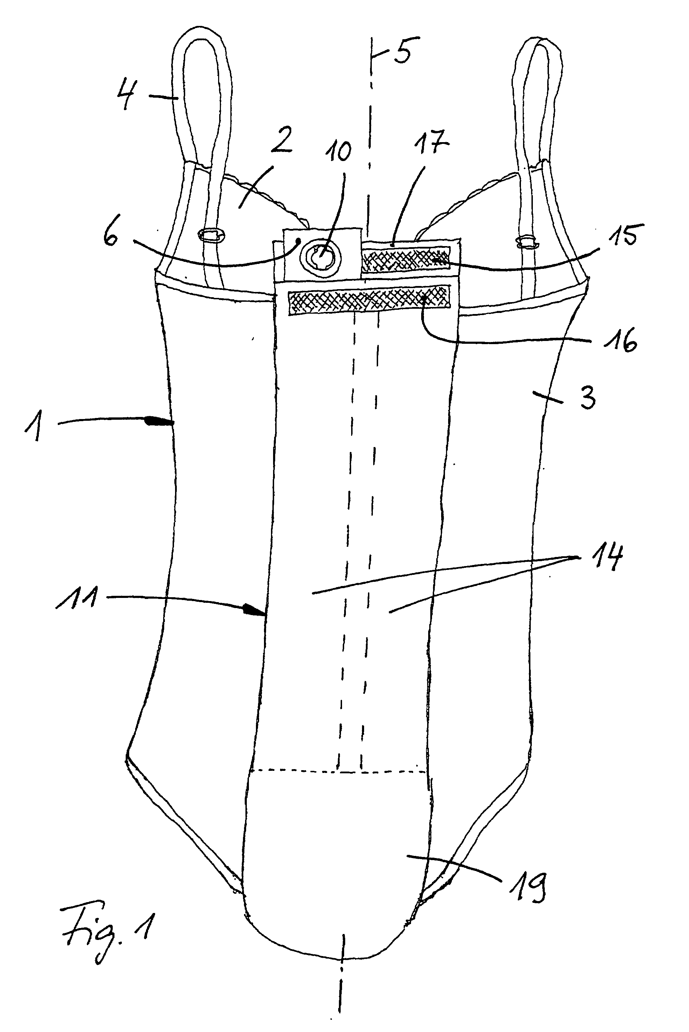

Fig. 1 zeigt ein Bekleidungsstück 1 in Form eines nur schematisch angedeuteten, aus einem flexiblen Material hergestellten Korseletts. Das Bekleidungsstück 1 weist ein Vorderteil 2 und ein mit diesem verbundenes Rückenteil 3 auf. In der Ansicht nach Fig. 1 ist das Bekleidungsstück 1 von innen nach außen gewendet und so dargestellt, daß insbesondere die Innenseite des Rückenteils sichtbar ist. Außerdem kann das Bekleidungsstück 1 mit üblichen Trägern 4 versehen sein. Im unteren Bereich sind das Vorderteil 2 und Rückenteil 3 zur Bildung eines Hosenteils unter Freilassung von Beinöffnungen miteinander verbunden.

Nach Fig. 1 ist auf der Innenseite des Rückenteils 3 zu beiden Seiten einer Mittellinie 5, die beim Tragen des Beldeidungsstücks 1 im wesentlichen längs der Wirbelsäule des Patienten verläuft, wenigstens je eine Luftkissenleiste 6 vorgesehen, die sich jeweils über diejenigen Bereiche des Rückenteils 3 erstreckt, die beim Tragen des Bekleidungsstück 1 dem Brustwirbelbereich und vorzugsweise auch dem oberen Lendenwirbelbereich des Patienten anliegen.

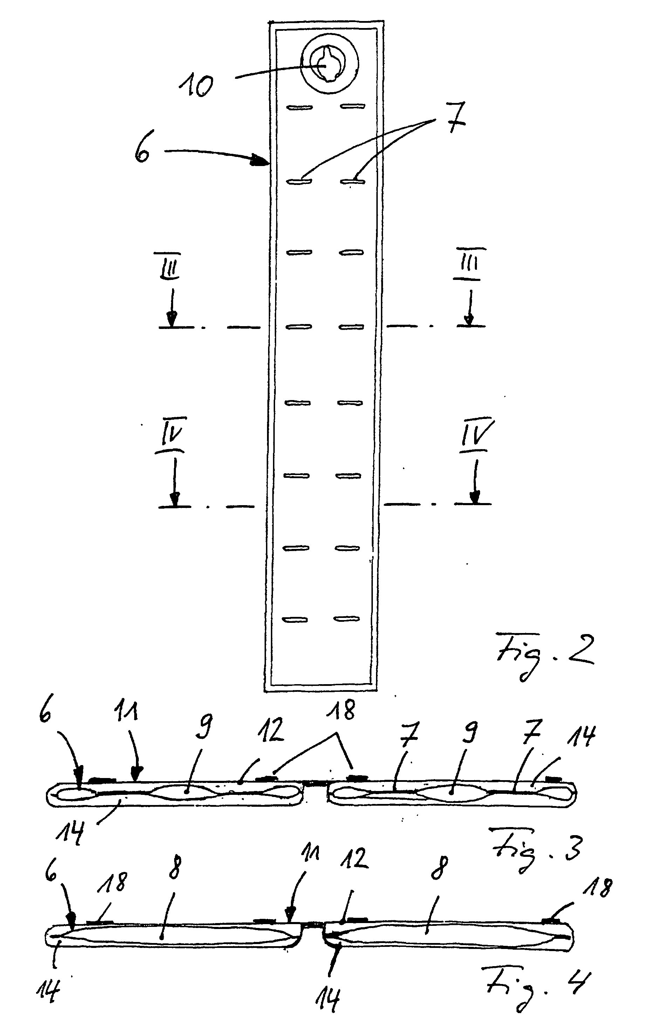

Nach Fig. 2 bis 4 besteht jede Luftkissenleiste 6 aus einem am Außenumfang geschlossenen Kissen mit im wesentlichen rechteckiger Kontur. Das Luftkissen ist durch eine Anzahl von Nähten 7, die z.B. parallel zu den kurzen Rechteckseiten verlaufen, in eine Mehrzahl von Luftkammern 8 unterteilt (Fig. 4), die in Richtung der langen Seiten hintereinander liegen und durch Unterbrechungen 9 (Fig. 3) der Nähte 7 hindurch sämtlich miteinander in Verbindung stehen. Außerdem weist jede Luftkissenleiste 6 an einer beim Tragen nicht störenden Stelle einen zum Aufblasen der Luftkissenleiste 6 bestimmten, mit einem Verschluß versehenen Anschlußstutzen 10 auf.

Zur Befestigung der Luftkissenleiste 6 am Rückenteil 3 dient vorzugsweise eine in Fig. 1, 3 und 4 grob schematisch angedeutete orthopädische Einrichtung 11. Diese enthält einen flexiblen Träger 12, der aus einem vorzugsweisen hautfreundlichen, textilen Material hergestellt ist und eine im wesentlichen rechteckige Kontur besitzt. Auf seiner vorderen Breitseite und beidseits einer zwischen den langen Rechteckseiten liegenden Mittellinie weist der Träger 12 zwei Taschen 14 auf, die eine der Größe der Luftkissenleisten 6 entsprechende Größe aufweisen und vorzugsweise in ihren oberen Enden mit Verschlußelementen 15, 16 versehen sind, die z.B. aus komplementären Klettverschlußstreifen bestehen, die an den Innenseiten von Taschenklappen 17 bzw. an den Außenseiten der Taschen 14 angebracht sind und beim Schließen der Taschen 14 in üblicher Weise zusammenwirken. Auf der hinteren Breitseite ist der Träger 12 mit Mitteln 18 (Fig. 3, 4) zu seiner Befestigung am Rückenteil 3 des Bekleidungsstücks 1 versehen. Diese Mittel 18 können ebenfalls aus Klettverschlußstreifen bestehen, die mit komplementären, an der Innenseite des Rückenteils 3 durch Nähen od. dgl. befestigten Klettverschlußstreifen zusammenwirken.

Zweckmäßig wird in die Taschen 14 bereits beim Hersteller je eine Luftkissenleiste 6 so eingesetzt, daß der Anschlußstutzen 10 von der Seite der Taschenklappen 17 her zugänglich ist. Auf diese Weise kann der Patient wählen, ob er ein fertiges, bereits mit den Luftkissenstreifen 6 versehenes Bekleidungsstück 1 oder nur die Einrichtung 11 erwerben und diese ggf. selbst in einem Bekleidungsstück 1 seiner Wahl befestigen möchte.

Beim Tragen des erfindungsgemäßen Bekleidungsstücks 1 werden die beiden Luftkissenleisten 6 beidseits der Brustwirbelsäule und ggf. zusätzlich beidseitig der oberen Lendenwirbelsäule massierend wirksam, wobei die Luftkissenleisten 6 durch das Bekleidungsstück 1 fixiert sind. Schon bei geringen Körperbewegungen führen die Luftkammern 8 durch ihre Massagewirkung zu einer verbesserten Durchblutung, wobei sich die Luft in den verschiedenen Luftkammern je nach Körperhaltung verschieben und dadurch der Anatomie des Benutzers anpassen kann. Das gilt insbesondere dann, wenn die Luftkissenleisten 6 nicht prall, sondern nur z. B. zu zwei Dritteln mit Luft gefüllt sind, so daß die Luft stets durch die Unterbrechungen 9 hindurch in andere Luftkammern 8 ausweichen kann, bei denen die Belastung geringer bzw. am geringsten ist. Dabei sollte außerdem das Bekleidungsstück 1 den üblichen Tragegewohnheiten eines Korseletts entsprechen und einen leichten, zirkulären, elastischen Druck auf den Körper ausüben bzw. über die Luftkammem 8 auf den Körper übertragen.

Zusätzlich kann vorgesehen sein, die Einrichtung 11 im unteren Bereich mit einer Tasche 19 für eine an sich bekannte Lumbosakral-Pelotte zu versehen, um auch deren Wirkung zu nutzen.

Die Erfindung bringt den überraschenden Vorteil mit sich, daß die Luftkissenleisten 6 anders als die Lumbosakral-Pelotten überwiegend im Sinne einer Durchblutungsförderung wirken. Sie unterstützen daher nicht nur die eingangs erläuterte Kombinationstherapie, sondern steigern den Übergang der zur Behandlung der Osteoporose verabreichten Substanzen über die Blutbahnen in die Knochen auch bereits bei vergleichsweise geringen körperlichen Bewegungen, d.h. ohne gezielte Gymnastik.

Die Erfindung ist nicht auf das beschriebene Ausführungsbeispiel beschränkt, das auf vielfache Weise abgewandelt werden könnte. Anstelle des beschriebenen Korseletts können beispielsweise Bekleidungsstücke in Form eines Body, eines einteiligen Badeanzugs, eines Hosenanzug, einer Hemdhose oder dergleichen vorgesehen werden, sofern diese so konfektioniert und/oder ausgebildet werden, daß sie die Luftkissenleisten 6 mit dem gewünschten geringen Druck an den Körper des Patienten drücken. Weiter ist klar, daß die Luftkissenleisten 6 auf andere als die beschriebene Weise an dem jeweiligen Bekleidungsstück 1 befestigt und/oder mit anderen als der beschriebenen Einrichtung 11 kombiniert werden können, die zum Beispiel auch zwei separate Taschen 14 ohne den gemeinsamen Träger 12 aufweisen könnte. Außerdem könnten die Luftkissenleisten 6 dauerhaft mit Luft gefüllt sein, in welchem Fall auf die Anschlußstutzen 10 (Ventile) verzichtet werden kann. Weiterhin könnten die am Körper zu liegen kommenden Innenflächen der Luftkissenleisten 6 oder der sie aufnehmenden Tasche(n) 14 mit Noppen oder anderen erhaben vorstehenden Mitteln versehen werden, um dadurch die mechanische Reizung der Mechano-/Propriorezeptoren zu erhöhen bzw. zu verstärken. Zur lokalen Verstärkung des einwirkenden Drucks der Luftkissenleisten 6 kann vorgesehen sein, auf der Rückseite des Bekleidungsstücks 1 die Enden von zwei oder mehr Gurten bzw. Zügeln zu befestigen, die nach dem Anlegen des Bekleidungsstücks 1 nach vom geführt, mehr oder weniger stark festgezogen und durch Klettverschlußelemente oder sonstwie untereinander verbunden werden. Die Enden dieser Zügel werden vorzugsweise etwa im Bereich der Mittellinie 5 und an der Außenseite des Bekleidungsstücks 1 befestigt. Weiter könnte pro Bekleidungsstück mehr als je eine Luftkissenleiste auf jeder Seite der Wirbelsäule angeordnet und/oder die Luftkissenleiste anders als dargestellt ausgebildet werden. Außerdem können alle Bekleidungsstücke und/oder Luftkissenleisten in unterschiedlichen Größen konfektioniert werden, um dadurch den unterschiedlichen Körpergrößen der Patienten Rechnung zu tragen. Schließlich versteht sich, daß die beschriebenen Merkmale auch in anderen als den dargestellten und beschriebenen Kombinationen verwendet werden können.

## Patentansprüche

1. Orthopädische Einrichtung zur Osteoporosebehandlung mit einem Vorderteil (2) und einem Rückenteil (3) aus einem flexiblen bzw. dehnbaren Material, wobei das Rückenteil an seiner Innenseite beidseitig einer gedachten, der Wirbelsäule eines Benutzers zugeordneten Mittellinie (5) mit wenigstens je einer Luftkissenleiste (6) versehen ist, die sich über den Brustwirbelbereich und gegebenenfalls auch den oberen Lendenwirbelbereich erstreckt und eine Mehrzahl von miteinander in Verbindung stehenden Luftkammern (8) aufweist.

2. Orthopädische Einrichtung nach Anspruch 1, **dadurch gekennzeichnet, daß** die Luftkissenleisten (6) in auf der Innenseite des Rückenteils (3) angebrachten Taschen (14) angeordnet sind.

3. Orthopädische Einrichtung nach einem der Ansprüche 1 bis 2, **dadurch gekennzeichnet, daß** die Taschen (14) an einem gemeinsamen Träger (12) vorgesehen und mit diesem am Rückenteil (3) befestigt sind.

4. Orthopädische Einrichtung nach einem der Ansprüche 1 bis 3, **dadurch gekennzeichnet, daß** die Befestigung der Taschen (14 ) oder des Trägers (12) am Rückenteil (3) durch Klettverschlußstreifen (18) erfolgt.

5. Orthopädische Einrichtung nach einem der Ansprüche 1 bis 4, **dadurch gekennzeichnet, daß** die Luftkammern (8) durch parallel zueinander verlaufende, unterbrochene Nähte (7) voneinander getrennt sind.

6. Orthopädische Einrichtung nach einem der Ansprüche 1 bis 5, **dadurch gekennzeichnet, daß** sie als Body, Korselett, Hemdhose, Hosenanzug oder einteiliger Badeanzug ausgebildet ist.

7. Orthopädische Einrichtung nach einem der Ansprüche 3 bis 6, **dadurch gekennzeichnet, daß** der Träger (12) flexibel ist, eine im wesentlichen rechteckförmige Kontur hat und auf einer vorderen Breitseite beidseits einer Mittellinie je eine zur Aufnahme einer Luftkissenleiste (6) bestimmte Tasche (14) und auf einer hinteren Breitseite Mittel (18) zu seiner Befestigung am Rückenteil (3) aufweist.

8. Orthopädische Einrichtung nach Anspruch 7, **dadurch gekennzeichnet, daß** die Taschen (14) mit Verschlußelementen (15,16) versehen sind.

9. Orthopädische Einrichtung nach einem der Ansprüche 7 und 8, **dadurch gekennzeichnet, daß** die Befestigungsmittel (18) und Verschlußelemente (15,16) Klettverschlußstreifen enthalten.

10. Orthopädische Einrichtung nach einem der Ansprüche 7 bis 9, **dadurch gekennzeichnet, daß** in jede Tasche (14) wenigstens eine Luftkissenleiste (6) eingesetzt ist.

## Claims

1. Orthopaedic device for the treatment of osteoporosis, having a front part (2) and a back part (3) formed from a flexible or stretchable material, the back part being provided on its inner side with at least one air cushion strip (6) on each side of an imaginary centre line (5) associated with the vertebral column of a user, said air cushion strips extending over the thoracic vertebral region and possibly also the upper lumbar vertebral region and having a plurality of air chambers (8) which communicate with one another.

2. Orthopaedic device according to claim 1, **characterised in that** the air cushion strips (6) are arranged in pockets (14) attached to the inner side of the back part (3).

3. Orthopaedic device according to one of claims 1 to 2, **characterised in that** the pockets (14) are provided on a common carrier (12) and secured with this to the back part (3).

4. Orthopaedic device according to one of claims 1 to 3, **characterised in that** the pockets (14) or the carrier (12) are secured to the back part (3) by Velcro strips (18).

5. Orthopaedic device according to one of claims 1 to 4, **characterised in that** the air chambers (8) are separated from one another by discontinuous seams (7) which extend parallel to one another.

6. Orthopaedic device according to one of claims 1 to 5, **characterised in that** it is designed as a body, corselet, combinations, trouser suit or one-piece swimming costume.

7. Orthopaedic device according to one of claims 3 to 6, **characterised in that** the carrier (12) is flexible, has a substantially rectangular contour and has on a broad front face, on each side of a centre line, a pocket (14) which is intended to receive an air cushion strip (6) and on a broad rear face means (18) for securing it to the back part (3).

8. Orthopaedic device according to claim 7, **characterised in that** the pockets (14) are provided with closure elements (15, 16).

9. Orthopaedic device according to one of claims 7 and 8, **characterised in that** the fastening means (18) and closure elements (15, 16) contain Velcro strips.

10. Orthopaedic device according to one of claims 7 to 9, **characterised in that** at least one air cushion strip (6) is inserted into each pocket (14).

## Revendications

1. Dispositif orthopédique pour le traitement de l'ostéoporose comprenant une partie avant (2) et une partie arrière ou de dos (3) réalisées dans une matière flexible ou extensible, dans lequel la face intérieure de la partie de dos (3) est munie, de part et d'autre d'une ligne médiane (5) imaginaire associée à la colonne vertébrale d'un utilisateur, d'au moins un bandeau de coussins d'air (6), qui s'étend au-dessus de la région des vertèbres dorsales et, le cas échéant aussi, au-dessus de la région supérieure des vertèbres lombaires et qui comporte une pluralité de chambres d'air (8) qui communiquent les unes avec les autres.

2. Dispositif orthopédique selon la revendication 1, **caractérisé en ce que** les bandeaux de coussins d'air (6) sont agencés dans des poches (14) réalisées sur la face intérieure de la partie arrière ou de dos (3).

3. Dispositif orthopédique selon l'une quelconque des revendications 1 à 2, **caractérisé en ce que** les poches (14) sont prévues sur un support (12) commun et sont fixées avec celui-ci sur la partie arrière ou de dos (3).

4. Dispositif orthopédique selon l'une quelconque des revendications 1 à 3, **caractérisé en ce que** la fixation des poches (14) ou du support (12) sur la partie de dos (3) est assurée par des bandes de fermeture auto-agrippantes (18).

5. Dispositif orthopédique selon l'une quelconque des revendications 1 à 4, **caractérisé en ce que** les chambres d'air (8) sont séparées les unes des autres par des coutures (7) interrompues, parallèles les unes aux autres.

6. Dispositif orthopédique selon l'une quelconque des revendications 1 à 5, **caractérisé en ce qu'**il est réalisé sous forme d'un body, d'un corset, d'un justaucorps, d'un tailleur-pantalon ou d'un maillot de bain une pièce.

7. Dispositif orthopédique selon l'une quelconque des revendications 3 à 6, **caractérisé en ce que** le support (12) est flexible, possède un contour essentiellement rectangulaire et comporte sur une face large avant, de part et d'autre d'une ligne médiane respectivement une poche (14), destinée à recevoir un bandeau de coussins d'air (6), et sur une face large arrière des moyens (18) pour le fixer contre la partie de dos (3).

8. Dispositif orthopédique selon la revendication 7, **caractérisé en ce que** les poches (14) sont munies d'éléments de fermeture (15, 16).

9. Dispositif orthopédique selon les revendications 7 et 8, **caractérisé en ce que** les moyens de fixation (18) et les éléments de fermeture (15, 16) comprennent des bandes de fermeture auto-agrippantes.

10. Dispositif orthopédique selon l'une quelconque des revendications 7 à 9, **caractérisé en ce qu'**au moins un bandeau de coussins d'air (6) est inséré dans chaque poche (14).
